# EUROPEAN PATENT SPECIFICATION

(11) **EP 2 451 520 B1**
(45) Date of publication and mention of the grant of the patent: **26.09.2018**
(21) Application number: 10797745.6
(22) Date of filing: 06.07.2010
(51) Int. Cl.: A61M 25/01, A61M 39/22, A61M 25/00

(54) **HUBS FOR TUBULAR DEVICES AND METHODS FOR MAKING THEM**
NABEN FÜR RÖHRENFÖRMIGE VORRICHTUNGEN SOWIE HERSTELLUNGSVERFAHREN DAFÜR
RACCORDS POUR DISPOSITIFS TUBULAIRES ET PROCÉDÉS POUR LES FABRIQUER

(30) Priority: 05.01.2010 US 292165 P; 06.07.2009 US 223352 P; 19.10.2009 US 581826; 13.08.2009 US 233803 P
(43) Date of publication of application: 16.05.2012
(73) Proprietor: Aust Development, LLC, Palo Alto, CA 94306 (US)
(72) Inventor: LEEFLANG, Stephen, A., Sunnyvale CA 94086 (US); EVERSULL, Christian, S., Palo Alto CA 94306 (US)
(74) Representative: Boult Wade Tennant LLP
(86) International application number: PCT/US2010/041109
(87) International publication number: WO 2011/005795

(56) References cited:
- DE-A1-102007 029 229
- US-A- 4 512 766
- US-A- 5 125 915
- US-A- 5 201 901
- US-A1- 2001 000 041
- US-A1- 2002 007 152
- US-A1- 2009 157 006
- US-B1- 6 290 668

## Description

### FIELD OF THE INVENTION

The present invention relates generally to apparatus and methods for delivering instruments and/or agents during a medical procedure, and, more particularly, to valves and/or hubs for guide sheaths, catheters, and other tubular devices for accessing body lumens and/or delivering instruments into body lumens of a patient, and to methods for making and using them.

### BACKGROUND

There are many medical procedures where a lead, catheter, electrode, and/or other medical device may be implanted into a patient's body cavity, recess, vessel, organ, and/or other body lumen. In many of these procedures, a delivery sheath, guide catheter, or other tubular member may be used to facilitate delivering the medical device, with the tubular member removed after placement of the medical device. Additionally, it may be desirable to provide a substantially fluid tight seal between the delivery sheath, guide catheter, or other tubular member and the lead, catheter, electrode, guidewire, and/or other medical device, e.g., for the purpose of hemostasis, infusion of therapeutic or diagnostic agents, and the like.

US2009/0157006 discloses an introducer sheath assembly having a sheath tube, a hub, a valve and a valve-retaining cap on the proximal end of the hub. The cap is so secured to the hub as to have a closed, locked position and also an open position permitting access to the interior of the hub and the valve while the cap remains secured to the hub.

DE10 2007 029 229 discloses a clamp adapter for a catheter that has a clamp body that receives an elastomeric clamp bushing. In order to fix a catheter inserted into the clamp bushing, the clamp bushing is axially compressed by a clamp lid. For this purpose, the clamp lid is moved axially on the clamp body with a clamp lever by means of a slotted guide.

However, the process of removing the tubular member from around the medical device after the medical device has been placed may be difficult and/or time consuming.

For example, a delivery sheath used to deliver a cardiac lead may not be easily removed from around the lead without disturbing the placement of the lead, which must remain in the patient. Therefore, an apparatus that may facilitate the delivery of devices, provide a seal or substantial seal, and/or facilitate removal without substantially disturbing placement of the lead and/or other device may be desirable.

### SUMMARY

According to the present invention, as claimed in claims 1, 10, there is provided a hub for a sheath, catheter, or other tubular device and a method for making a sheath, catheter, or other tubular device as claimed in the appended claims.

The present invention is directed generally to apparatus and exemplary methods for delivering instruments and/or agents during a medical procedure. More particularly, the present invention is related to valves and/or hubs for guide sheaths, catheters, and other tubular devices for accessing and/or delivering instruments into body lumens of a patient, and to methods for making and using them.

In accordance with the invention, a hub is provided for a sheath, catheter, or other tubular device that includes a first hub portion including a first hub lumen sized for receiving a medical device therethrough, and a second hub portion including a second hub lumen, the second hub portion coupled to the first hub portion such that the first and second hub lumens are aligned with one another and the first and second hub portions are spaced apart from one another to define a gap for receiving a valve therein.

The second portion is coupled to the first hub portion by a hub arm. For example, the hub arm may be sufficiently flexible such that the first hub portion may be directed away from the second hub portion to increase a size of the gap to accommodate inserting the valve within the gap and/or that may be resiliently biased to return towards its original shape to capture a valve within the gap. In addition or alternatively, the first and/or second valve portions may include one or more connectors for securing the valve within the gap. Optionally, the hub arm may include a side port including an opening that communicates with the second hub lumen, e.g., for delivering fluid into the second hub lumen.

The hub includes a valve secured within the gap between the first and second hub portions. The valve includes a valve passage therethrough, e.g., for accommodating receiving a medical device through the first and second hub lumens, while providing a substantially fluid tight seal. For example, the valve passage may include a bore extending partially from a first end of the valve towards a second end of the valve, and a slit that extends from the bore to the second end of the valve. The bore may have a circular or other cross-section, e.g., to accommodate introducing a medical device therethrough, and/or the slit may have opposing slit regions that are biased to close against one another, e.g., to provide a fluid- tight seal, and that are separable to accommodate receiving the medical device introduced through the bore.

In accordance with another arrangement, a valve is provided for a hub of a sheath, catheter, or other tubular device that includes a valve body including a first end, a second end, and an outer surface extending between the first and second ends; and a valve passage extending through the valve body between the first and second ends. In an exemplary embodiment, the valve passage may include a bore extending partially through the valve body from the first end towards the second end, and a slit extending through the valve body from the bore to the second end. The bore may have a circular or other cross-section, e.g., to accommodate introducing a medical device therethrough, and/or the slit may have opposing slit regions that are biased to close against one another, e.g., to provide a fluid-tight seal, and that are separable to accommodate receiving the medical device introduced through the bore. Optionally, the valve passage may include a recess within the bore that has a larger diameter or other cross-section than the bore, e.g., for receiving a lubricant and/or facilitating expansion of the valve when a medical device is inserted through the valve passage.

Optionally, the valve may include one or more connectors for securing the valve body to a hub of a tubular device, e.g., at least one of a groove and a beveled edge on the first end of the valve body. The one or more connectors may extend circumferentially around the first and second ends of the valve body, e.g., similar to corresponding connectors on the hub to which the valve is secured. In alternative embodiments, the connectors may include undersized annular flanges extending from the valve body that are sized to be received in respective openings in a hub, or oversized annular flanges that are sized to be received over respective openings in a hub.

In accordance with still another arrangement, a valve is provided for a hub of a sheath, catheter, or other tubular device that includes a valve body including a first end, a second end, and one or more connectors on the first and second ends for securing the valve body to a hub of a tubular device; and a valve passage extending through the valve body between the first and second ends. The valve passage may include a bore extending partially through the valve body from the first end towards the second end, and a slit extending through the valve body from the bore to the second end. The valve body may be resiliently flexible such that a cross-section of the bore may be increased to accommodate receiving a medical device through the bore that has a cross- section larger than the bore while maintaining a substantially fluid tight seal around the medical device, and/or the slit may include opposing slit regions, e.g., having substantially planar opposing surfaces defining the slit that open to accommodate receiving a medical device through the valve passage yet resiliently close against one another when the medical device is removed to maintain a substantially fluid tight seal through the valve passage.

In accordance with yet another arrangement, a valve is provided for a hub of a sheath, catheter, or other tubular device that includes a valve body including a first end, a second end, and one or more connectors on the first and second ends for securing the valve body to a hub of a tubular device; a valve passage extending through the valve body between the first and second ends, the valve passage including a bore extending partially through the valve body from the first end towards the second end, and a slit extending through the valve body from the bore to the second end; and a lubricant within the bore to reduce friction when a medical device is inserted through the valve passage. The valve body may be resiliently flexible such that a cross-section of the bore may be increased to accommodate receiving a medical device through the bore that has a cross-section larger than the bore while maintaining a substantially fluid tight seal around the medical device, and/or the slit may include opposing slit regions that open to accommodate receiving a medical device through the valve passage yet resiliently close when the medical device is removed to maintain a substantially fluid tight seal through the valve passage.

In an exemplary arrangement, the bore may include a first bore region having a first diameter or other cross-section extending from the first end to an intermediate location in the valve body, and a second bore region at the intermediate location, e.g., at the bottom of the bore, that has a larger diameter or other cross-section than the first bore region, and wherein the lubricant is disposed within the second bore region.

In accordance with still another arrangement, a method is provided for making a valve for a sheath, catheter, or other tubular device that includes forming a valve body including a first end, a second end, and a bore extending partially through the valve body from the first end towards the second end; and inserting an instrument into the bore to create a slit extending from the end of the bore to the second end.

In accordance with the invention a method is provided for making a sheath, catheter, or other tubular member that includes forming a hub including a first hub portion including a first hub lumen and a second hub portion including a second hub lumen, the second hub portion coupled to the first hub portion such that the first and second hub lumens are aligned with one another and the first and second hub portions are spaced apart from one another to define a gap; forming a valve body including a valve passage extending therethrough; and securing the valve body within the gap between the first and second hub portions such that the valve passage accommodates receiving a medical device therethrough when the medical device is introduced through the first and second hub lumens of the first and second hub portions while providing a substantially fluid tight seal. In addition, a tubular body may be attached to the second hub portion such that a lumen of the tubular body communicates with the first and second hub lumens.

In accordance with still another arrangement, a method is provided for making a sheath, catheter, or other tubular member that includes forming a handle comprising a first handle portion with a first hub portion including a first hub lumen and a second handle portion with a second hub portion including a second hub lumen, the first and second handle portions coupled together opposite the hub portions such that the first and second hub lumens are aligned with one another and the first and second hub portions are spaced apart from one another to define a gap; selecting a valve body comprising a valve passage extending therethrough; directing the first and second hub portions away from one another to increase a size of the gap; inserting the valve body within the gap between the first and second hub portions; and directing the first and second hub portions towards one another to capture the valve body between the first and second hub portions.

The apparatus may be used in an exemplary method for delivering a medical device into a body lumen within a patient's body. Initially, a distal end of a tubular device may be introduced into the patient's body, the tubular device including a hub on a proximal end thereof that includes a valve secured between a proximal hub portion and a distal hub portion. The distal end of the tubular device may be positioned within a body lumen, and a medical device may be inserted through the proximal hub portion, the valve, and the distal hub portion until a distal end of the medical device is positioned within the body lumen. The proximal hub portion, valve, distal hub portion, and tubular device may be sequentially cut to remove the tubular device from around the medical device while the medical device distal end remains within the body lumen.

Other aspects and features of the present invention will become apparent from consideration of the following description taken in conjunction with the accompanying drawings.

### BRIEF DESCRIPTION OF THE DRAWINGS

The drawings illustrate exemplary embodiments of the invention, in which:
FIG. 1A is a perspective view of an exemplary embodiment of a tubular device, including a hub on its proximal end and a valve connectable to the hub.
FIG. 1B is a cross-sectional view of the proximal end of the tubular device of FIG. 1A, taken along line 1B-1B, before the valve has been coupled to the hub.
FIG. 2A is an end view of an exemplary embodiment of a valve that may be coupled to the hub of FIGS. 1A and 1B.
FIG. 2B is a cross-sectional side view of the valve of FIG. 2A, taken along line 2B-2B.
FIG. 2C is a cross-sectional top view of the valve of FIGS. 2A and 2B, taken along line 2C-2C.
FIG. 2D is a cross-sectional top view of an alternative embodiment of the valve of FIGS. 2A-2C.
FIG. 3 is a cross-sectional detail of the hub of FIGS. 1A and 1B, showing the valve of FIGS. 2A-2C coupled to the hub.
FIGS. 4A-4C are cross-sectional views of a valve body showing a method for making a slit through the valve body to create the valve of FIGS. 2A-2C.
FIG. 5A is a side view of another exemplary embodiment of a hub including a valve coupled to the hub that includes undersized flanged connectors.
FIG. 5B is a side view detail of the valve of FIG. 5A.
FIG. 6A is a side view of still another exemplary embodiment of a hub including a valve coupled to the hub that includes oversized flanged connectors.
FIG. 6B is a side view detail of the valve of FIG. 5A.
FIGS. 7A-7C are side views of alternative embodiments of handles that may be provided for receiving a valve.
FIGS. 8A-8C are side views of yet another embodiment of a handle showing a method for securing a valve to the handle.
FIGS. 9A and 9B are side views of still another embodiment of a handle showing a method for securing a valve to the handle.
FIGS. 10A and 10B are details of the handle of FIGS. 9A and 9B.
FIG. 11 is a side view of yet another embodiment of a handle for receiving a valve.

### DETAILED DESCRIPTION OF THE EXEMPLARY EMBODIMENTS

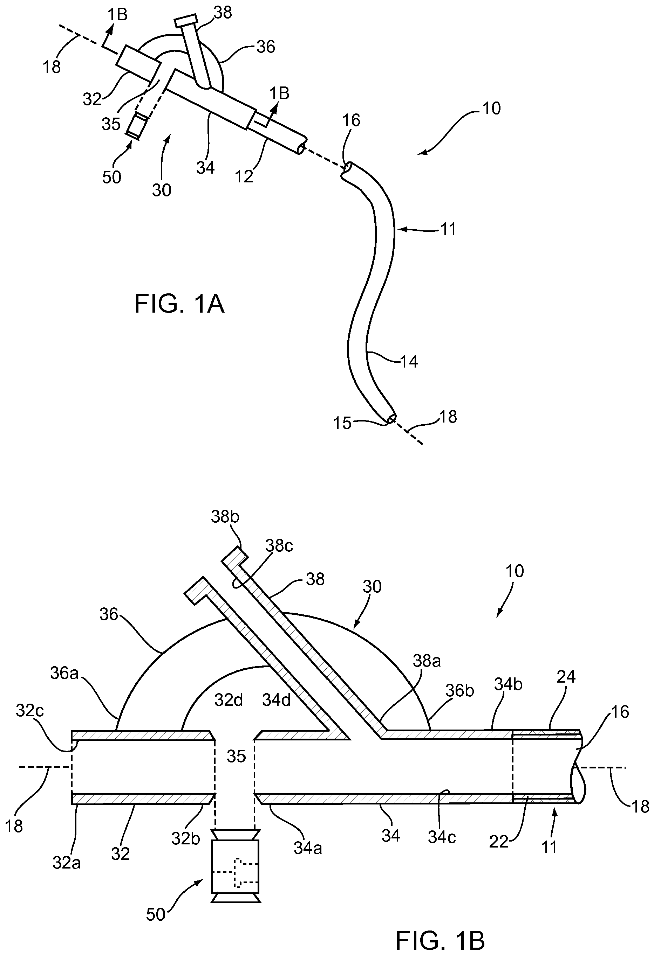

Turning to the drawings, FIGS. 1A and 1B show an exemplary embodiment of an apparatus 10 for accessing a body lumen (not shown) and/or for delivering one or more fluids, agents, and/or instruments (also not shown) within a body lumen. In exemplary embodiments, the apparatus 10 may be a guide catheter, a procedure catheter, a sheath, an imaging device, or other tubular device sized for introduction into a body lumen, such as a vessel within a patient's vasculature, a passage within a patient's gastrointestinal tract, urogenital tract, reproductive tract, respiratory tract, lymphatic system, and the like.

Generally, the apparatus 10 includes an elongate tubular body 11 including a proximal end 12, a distal end 14 sized for introduction into a body lumen, a lumen 16 extending between the proximal and distal ends 12, 14 along a central longitudinal axis 18, and a handle or hub 30 on the proximal end 12 including a valve 50 for allowing one or more devices to be introduced into the lumen 16. Optionally, the apparatus 10 may include one or more additional lumens (not shown), which may be disposed concentrically around, side-by-side with, or otherwise adjacent the lumen 16. The lumen 16 may be sized for receiving a guide wire, procedure catheter, cardiac lead, needle, or other instrument (not shown), and/or for delivering fluids or other flowable agents or materials therethrough, as described further below.

As can be seen in FIG. 1B, the tubular body 11 may be constructed from one or more layers, e.g., an inner liner 22 surrounding the lumen 16, a reinforcing layer surrounding the inner liner (not shown), and an outer layer 24. Optionally, one or more coatings (not shown) may be applied to the inner surface of the inner liner 22. In an exemplary embodiment, a hydrophilic coating, such as Polyvinylpyrrolidone, may be sprayed or otherwise applied onto the surface of the inner liner 22 during fabrication to provide a lubricious inner surface for the lumen 16 of the tubular body 11.

The layers of the tubular body 11 may be attached to one another, e.g., by laminating, adhering, adhesive bonding, ultrasonic welding, reflowing or other heating, and the like. The construction of the tubular body 11 may be substantially uniform or may vary between the proximal and distal ends 12, 14, e.g., by varying the inner liner, 22, reinforcing layer, and/or outer layer 24 along the length of the tubular body 11. Optionally, the inner liner 22, reinforcing layer, and/or outer layer 24 may include one or more sublayers (not shown), which may vary in construction in various portions of the tubular body 11.

In one exemplary embodiment, the proximal end 12 may be substantially rigid or semi-rigid, e.g., providing sufficient column strength to allow the tubular body 11 to be pushed from the proximal end 12, while the distal end 14 may be substantially flexible or semi-rigid. Thus, the distal end 14 of the tubular body 11 may be advanced or otherwise manipulated within a patient's body from the hub 30 and/or proximal end 12 without substantial risk of buckling and/or kinking.

In exemplary embodiments, the tubular body 11 may have an outer diameter between about half and twenty millimeters (0.5-20 mm) or between about one and five millimeters (1-5 mm), and a length between about five and one hundred fifty centimeters (5-150 cm). The inner liner 22 may have a wall thickness between about 0.0001-0.01 inch (0.0025-0.25 mm) and the outer layer 24 may have a wall thickness between about 0.0005-0.2 inch (0.0127-5.08 mm).

The outer layer 22 may have a substantially homogenous construction between the proximal and distal ends 12, 14. Alternatively, the construction may vary along the length of the apparatus 10 to provide desired properties. For example, the outer layer 22a at or adjacent the proximal end 12 may be substantially rigid or semi-rigid, e.g., providing sufficient column strength to facilitate the apparatus 10 being pushed from the proximal end 12. In addition, the reinforcing layer or other material in the outer layer 22 may allow the apparatus 10 to be twisted from the proximal end 12, e.g., to rotate the distal end 14 within a patient's body. Thus, the distal end 14 of the apparatus 10 may be manipulated within a patient's body from the proximal end 12 without substantial risk of buckling and/or kinking. Optionally, the outer layer 22b at or adjacent the distal end 14 may be substantially flexible or semi-rigid, e.g., to allow the distal end 14 to bend easily or otherwise be advanced through tortuous anatomy and/or provide a substantially atraumatic distal tip 15. Furthermore, the outer layer 22a, may have one or more transition regions along its length, transitioning from one desired construction to another. Exemplary outer layers that may be included in the apparatus 10 and methods for making them are disclosed in U.S. Patent Nos. 4,478,898, 4,863,442, 5,217,440, 5,254,107, 5,676,659, 5,811,043, 5,836,926, 6,004,310, 6,669,886, 6,837,890, and 6,945,970.

Optionally, the distal end 14 may include a tapered, rounded, or otherwise shaped distal tip 15, e.g., to provide a substantially atraumatic tip and/or to facilitate advancement or navigation through various anatomy. In addition or alternatively, the distal end 14 may include one or more therapeutic and/or diagnostic elements, e.g., one or more balloons, stents, sensors, electrodes, steering mechanisms, imaging devices, needles, and the like (not shown), depending upon the particular intended application for the apparatus 10.

Returning to FIG. 1B, an inventive hub 30 (which may be any of the embodiments described herein) is shown that includes a first or proximal hub portion 32 and a second or distal hub portion 34 coupled together by a hub arm 36. The first hub portion 32 is an elongate tubular body including a first end 32a, a second end 32b, and a first hub lumen 32c extending therebetween. Similarly, the second hub portion 34 is an elongate tubular body including a first end 34a, a second end 34b, and a second hub lumen 34c extending therebetween. The lengths of the first and second hub portions 32, 34 may substantially the same or different than one another, e.g., between about 0.1 and ten centimeters (0.1-10 cm).

The hub portions 32, 34 may have a substantially uniform wall thickness. Alternatively, the thickness of the hub portions 32, 34 may vary around a circumference of the hub portions 32, 34. For example, the hub portions 32, 34 may include a relatively thin or weakened region (not shown) extending axially along the hub portions 32, 34, e.g., to facilitate slitting the hub 30 during use, as explained further below. In an exemplary embodiment, the relatively thin region may be disposed generally opposite the hub arm 36, e.g., such that the relatively thin region may be slit or cut without substantial interference from the hub arm 36.

The first and second hub portions 32, 34 are aligned with one another such that the first and second hub lumens 32c, 34c are also aligned with one another, e.g., concentrically around the longitudinal axis 18 of the apparatus 10. The hub lumens 32c, 34c is sized to accommodate slidably receiving a medical device therethrough, e.g., having a diameter between about one and ten millimeters (1-10 mm), which may allow introduction of a medical device through the hub 30 into the tubular body 11, as explained further below. Alternatively, the hub portions 32, 34 and/or hub lumens 32c, 34c may have an elliptical or other cross-section rather than a circular cross- section, if desired.

The first end 32a of the first hub portion 32 may include a transition and/or other features to facilitate introducing a medical device into the first hub lumen 32c. For example, the first end 32a may include a tapered wall (not shown) communicating with the first hub lumen 32c. The second end 34b of the second hub portion 34 may include one or more connectors (not shown) for attaching the tubular body 11 to the hub 50. For example, the second end 34b may include one or more tabs, slots, threads, and the like (not shown) that may be engaged with complementary slots, tabs, threads, and the like (also not shown) on the proximal end 12 of the tubular body 11. In addition or alternatively, the second end 34b may be engaged with the proximal end 12 by an interference fit, may be attached by bonding with adhesive, fusing, sonic welding, heat bonding, reflowing, insert molding, and the like, if desired.

As shown in FIG. IB, the arm 36 includes a first end 36a attached to the first hub portion 32 and a second end 36b attached to the second hub portion 34, thereby maintaining the first and second hub portions 32, 34 spaced apart from one another to define a gap 35. In an exemplary embodiment, the hub portions 32, 34 may be spaced apart such that the gap 35 has an axial length (substantially parallel to the longitudinal axis 18) or other size, e.g., between about one and ten millimeters (1-10 mm). The arm 36 may be sufficiently flexible such that the first hub portion 32 may be directed away from the second hub portion 34 to increase an axial length or other size of the gap 35, e.g., to accommodate inserting the valve 50 within the gap 35, as explained further below.

For example, during manufacturing or otherwise before use, the hub portions 32, 34 may be directed slightly apart from one another to increase the length of the gap 35 while a valve 50 is inserted within the gap 35. When the hub portions 32, 34 are released, the hub portions 32, 34 may return to their original position, e.g., engaging or otherwise capturing the valve 50 within the gap 35, as explained further below. As shown in FIG. 1B, the arm 36 may have an arcuate shape, e.g., a curved shape having a substantially uniform or varying radius of curvature between the first and second ends 36a, 36b. The shape and/or material of the arm 36 may accommodate bending the arm 36 open slightly when the hub portions 32, 34 are directed apart, and automatically returning the arm 36 to its original shape when the hub portions 32, 34 are released, thereby returning the hub portions 32, 34 to their original relative positions. Alternatively, the arm 36 may have other shapes, which may be flexible to accommodate inserting the valve 50 within the gap 35.

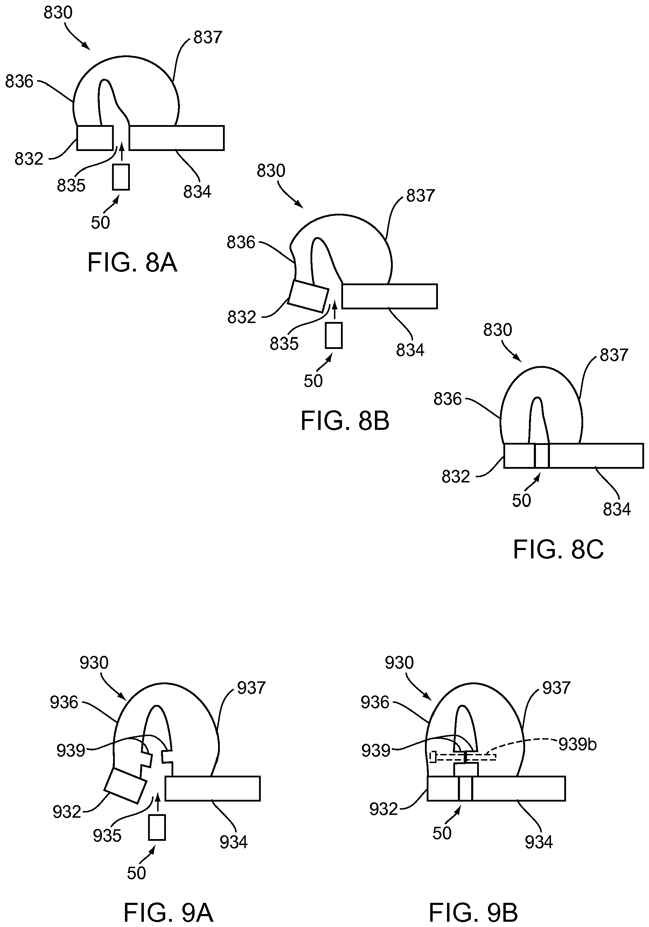

For example, turning to FIGS. 8A-8C, another embodiment of a hub 830 is shown that includes a proximal or first handle portion 836 coupled to a distal or second handle portion 847. The first handle portion 836 includes a first hub portion 832 and the second handle portion 837 includes a second hub portion 834 that are spaced apart when the hub 830 is free from external forces to define a gap 835, as shown in FIG. 8A. The first handle portion 836 may be sufficiently flexible that the first handle portion 836 may be bent to direct the first hub portion 832 away from the second hub portion 834 and open the gap 835. For example, as shown in FIG. 8B, the first handle portion 836 may be relatively narrower in cross-section than the second handle portion 837 to provide sufficient flexibility to resiliently bend the first handle portion 836. Once a valve 50 is inserted into the expanded gap 835 and/or attached to one of the hub portions 832, 834, the first handle portion 836 may be released and/or otherwise allowed to resiliently return to its original position, thereby capturing the valve 50 between the hub portions 832, 834, as shown in FIG. 8C.

Turning to FIGS. 9A and 9B, an alternative embodiment of a hub 930 is shown that includes first and second handle portions 936, 937 with respective hub portions 932, 934. In this alternative, the handle portions 936, 937 may be substantially rigid but may be pivotally coupled together, e.g., opposite the hub portions 932, 934, such that the handle portions 936, 937 may be opened or closed to increase or decrease a gap 935 between the hub portions 936, 937. Unlike the previous embodiments, the handle portions 936, 937 include opposing stops or other features 939a that are configured to limit movement of the handle portions 936, 937 to an orientation providing the desired gap 935.

For example, as shown in FIG. 9A, the hub 930 may be provided initially with the stops 939a spaced apart from one another such that the gap 935 is larger than the size for the selected valve 50. Once the vale 50 is positioned within the gap 935 and/or attached to one of the hub portions 932, 934, the first handle portion 936 may be pivoted towards the second handle portion 937 until the stops 939a abut or otherwise contact one another to capture the valve 50 in the final gap 935. The handle portions 936, 937 may be plastically deformed when pivoted to close the gap 935, e.g., such that the handle portions 936, 937 remain in the final orientation shown in FIG. 9B. In addition or alternatively, one or more fasteners 939b may be coupled between the handle portions 936, 937, e.g., through the stops 939a, as shown. In addition or alternatively, the stops 939a may be attached together by bonding with adhesive, fusing, sonic welding, cooperating detents or other connectors, and the like (not shown). Optionally, the valve 50 may also be attached to one or both hub portions 932, 934, e.g., similar to other embodiments herein.

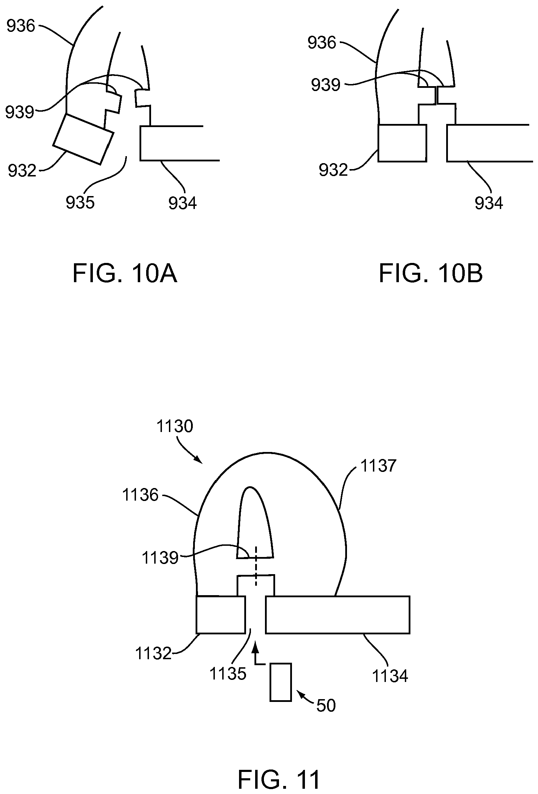

Turning to FIGS. 10A and 10B, the opposing surfaces of the hub portions 932, 934 defining the gap 935 may not be substantially parallel to one another when the gap 935 is opened, as shown in FIG. 10A. The angle of the opposing surfaces may correspond generally to the degree that the first handle portion 936 is rotated when the gap 935 is closed, e.g., such that the opposing surfaces are substantially parallel to one another or are otherwise configured to capture the valve 50 when the stops 939 engage one another, as shown in FIG. 10B.

Turning to FIG. 11, yet another embodiment of a hub 1130 is shown that includes first and second handle portions 1136, 1137 pivotally coupled to one another, e.g., generally opposite a gap 1135 between hub portions 1132, 1134 on respective handle portions 1136, 1137. The hub 1130 also includes a bridge feature 1139 extending between the handle portions 1136, 1137, e.g., adjacent the gap 1135. For example, the hub 1130 may be molded or otherwise integrally formed as a single piece including the handle portions 1136, 1137, hub portions 1132, 1134, and bridge 1139.

To accommodate capturing a valve 50, the bridge 1139 may be cut or otherwise severed to create stops, thereby allowing the handle portions 1136, 1137 to pivot away from one another (similar to FIG. 9A) to increase the size of the gap 1135. With the gap 1135 opened, the valve 50 may be positioned in the gap 1135 and/or attached to one of the hub portions 1132, 1134. The handle portions 1136, 1137 may then be released or otherwise returned to their original orientation. For example, the pivot point of the handle portions 1136, 1137 may be biased to return towards its original configuration or the handle portions 1136, 1137 may be plastically deformed to close the gap 1135 and capture the valve 50, e.g., when the stops 1139 contact one another. In addition, if desired, one or more fasteners 1139b may be directed through the stops 1139 or otherwise between the first and second handle portions 1136, 1137 to secure the hub 1130 with the valve 50 captured between the hub portions 1132, 1134. In addition or alternatively, the stops 1139 and/or the first and second handle portions 1136, 1137 may be attached together by bonding with adhesive, fusing, sonic welding, cooperating detents or other connectors, and the like (not shown). Optionally, the valve 50 may also be secured to the hub portions 1132, 1134, e.g., by bonding with adhesive, fusing, sonic welding, and the like in addition or instead of the fastener(s).

In a further alternative, with general reference to FIG. 1B, the arm 36 and/or hub 30 may be substantially rigid and/or the gap 35 substantially fixed. In this alternative, the hub or handle may be formed as separate components that are attached together when a valve is being secured to the handle, as described further below.

In addition or in a further alternative, e.g., where the hub 30 is substantially rigid, the valve 50 may be inserted into the gap 35 by temporarily deforming the valve 50. The valve 50 may then return to its original shape, thereafter being engaged within the gap 35. The valve 50 may be introduced into the gap 35 from the side or through the hub lumen 32c or 34c of the hub portion 32 or 34. Optionally, if desired, the arm 36 may have other shapes adapted to enable secure gripping of the arm 36 for manipulation of the apparatus 10, including, for example, advancing, retracting or rotating within a body lumen, and/or removal of the apparatus 10 by slitting.

Optionally, the second end 32b of the first hub portion 32 and/or the first end 34a of the second hub portion 34 may include one or more connectors (not shown) for cooperating with complementary connectors on the valve 50 and/or for otherwise engaging the valve 50 captured within the gap 35. For example, the ends 32b and/or 34a may include one or more tabs, slots, threads, features and the like (not shown), which may be engaged with complementary slots, tabs, threads, features and the like (also not shown) on the valve 50. Alternatively, or in addition, as shown in FIG. 1B, the ends 32b, 34a may include beveled edges 32d, 34d extending around a circumference of the ends 32b, 34a that may be captured in corresponding slots 58 in the valve 50, as explained further below.

Returning to FIG. 1B, the hub 30 (or any of the other hubs or handles described herein) may also include one or more side ports, e.g., a first side port 38 communicating with the lumen 16. Optionally, one or more additional side ports (not shown) may be provided on the hub 30 communicating with respective lumen(s), e.g., if the tubular body 11 includes an inflation lumen for a balloon on the distal end 14 (also not shown). The side port 38 generally includes a tubular body including a first end 38a coupled to the second hub portion 34, a second end 38b including a connector (not shown), and a lumen 38c extending from the second end 38b to the first end 38a and communicating with the second hub lumen 34c. In an exemplary embodiment, the connector on the second end 38b may include a luer lock connector, a hemostatic seal, and the like, e.g., for coupling a source of fluid, inflation media, and/or vacuum to the side port 38.

Optionally, the hub 30 may include one or more other connectors, e.g., luer lock connectors, electrical connectors, and the like (not shown), for connecting other devices (not shown) to the apparatus 10, such as syringes, displays, controllers, and the like (also not shown). In addition, the hub 30 may include one or more actuators, such as sliders, buttons, switches, and the like, e.g., for activating and/or manipulating components (also not shown) on the distal end 14 or otherwise operating the apparatus 10.

Multiple components of the hub 30 may be integrally formed together as a single piece or may be formed separately and then attached together to provide the hub 30. For example, the hub portions 32, 34, arm 36, and side port 38 may be formed as a single piece, e.g., by injection molding, casting, and the like. Alternatively, as described further elsewhere herein, the hub portions 32, 34 and side port 38 may be formed separately, e.g., by extrusion, injection molding, casting, and the like, and attached to the hub arm 36 and/or to each other, as desired, e.g., using cooperating connectors (not shown), bonding with adhesive, fusing, sonic welding, heat bonding, reflowing, insert molding, and the like. The hub 30 and/or its components may be formed from plastic, metal, or composite materials, as desired, such as nylon, PEBAX, PTFE, HDPE, and the like.

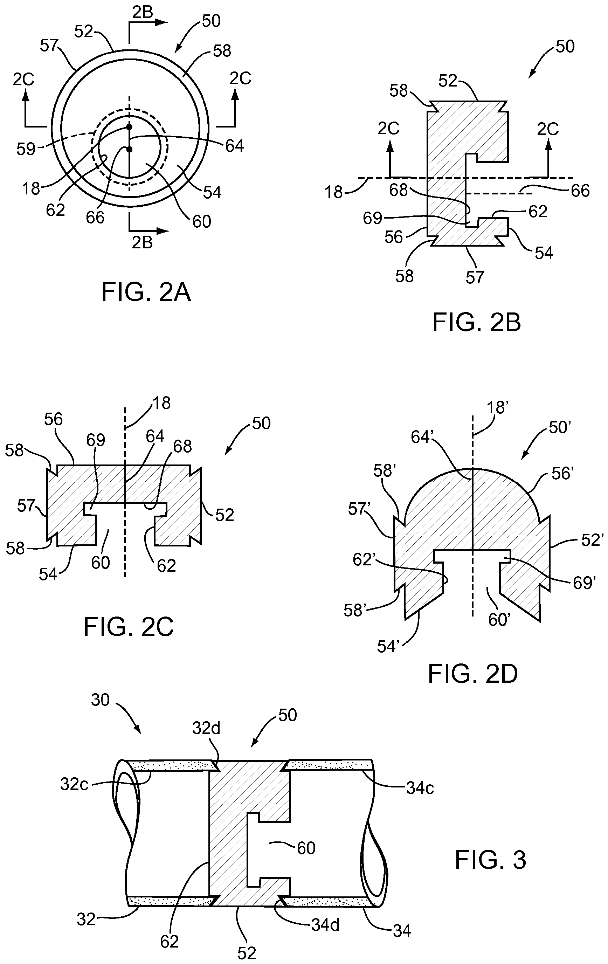

Turning to FIGS. 2A-2C, an exemplary embodiment of a valve 50 is shown that generally includes a valve body 52 including a proximal or first end 54, a distal or second end 56, an outer surface 57 extending between the first and second ends 54, 56, and a valve passage 60 extending between the first and second ends 54, 56. As shown, the valve passage 60 includes a bore 62 and a slit 64 that are formed adjacent one another within the valve body 52. For example, the bore 62 may extend from the first end 54 partially through the valve body 52 to an intermediate region such that the bore 62 defines a bottom surface 68, and the slit 64 may extend from the bore 62, e.g., from the bottom surface 68, to the second end 56 of the valve body 52. As explained further below, the bore 62 may allow the valve 50 to provide a substantially fluid-tight seal when a medical device (not shown) is inserted into the valve passage 60, while the slit 64 may provide a fluid-tight seal when the valve passage 60 is empty (i.e., without a medical device inserted into the valve passage 60).

Optionally, the valve passage 60 may also include a recess 69, e.g., at the bottom 68 of the bore 62. For example, the recess 69 may extend circumferentially around the bore 62 adjacent the bottom 68 to define an annular pocket within the valve passage 60 that has a diameter or other cross-section that is larger than the bore 62. For example, with the valve body 52 in a relaxed state (e.g., without a medical device, tool, or other instrument inserted into the bore 62), the bore 62 may have a diameter between about 0.25 and eight millimeters (0.25-8 mm), and the recess 69 may a diameter larger than the bore 62 between about 0.3 and nine millimeters (0.3-9 mm). Alternatively, one or more discrete recesses or pockets (not shown) may be provided within the bore 62, e.g., spaced apart from one another circumferentially around the bore 62 and/or axially along the bore 62, if desired. Such recess(es) may accommodate providing a lubricant within the valve passage 60, e.g., to reduce friction or otherwise facilitate introducing a medical device, tool, or other instrument into the valve passage 60, as explained further below. In addition or alternatively, the recess 69 may facilitate expansion of the bore 62, e.g., when a medical device, tool, or other instrument is inserted into the bore 62, also as explained further below. In addition or alternatively, the recess 69 may be adapted decrease or stop propagation of tears during expansion of the bore 62 or slit 64, e.g., when a medical device, tool, or other instrument is inserted through the valve body 52, as described further below.

As best seen in FIGS. 2B and 2C, the valve body 52 may be formed such that both the outer surface 57 and bore 62 extend substantially parallel to the longitudinal axis 18. However, as best seen in FIGS. 2A and 2B, a central axis 66 of the bore 62 may be offset from the longitudinal axis 18, e.g., such that the bore 62 is closer to the outer surface 57 of the valve body 52 on one side than the opposite side. Thus, a side wall region of the valve body 52 adjacent the bore 62 may be thinner on one side of the valve body 52 than other side wall regions, which may facilitate slitting the valve 50 during use, as explained further below.

The bore 62 may be sized appropriately to allow a medical device (not shown) to pass freely through the bore 62 without substantial frictional resistance and/or to provide a seal around the medical device to prevent substantial fluid leakage when the medical device is passed through the bore 62. Optionally, the valve body 52 may be resiliently flexible such that the bore 62 may be dilated or otherwise expanded when a medical device is inserted into the bore 62 and may resiliently return to its original size when the medical device is removed. Thus, the bore 62 may expand to accommodate a medical device having a larger cross-section than the bore 62 with the valve body 52 in the relaxed state. For example, with the valve body 52 in the relaxed state (e.g., without a medical device inserted into the bore 62), the bore 62 may have a diameter between about 0.25 and eight millimeters (0.25-8 mm), but may be expandable to larger diameters, e.g., between about 0.35 and ten millimeters (0.35-10 mm).

The slit 64 may also extend substantially parallel to the longitudinal axis 18 from the bottom 68 of the bore 62 to the second end 56 of the valve body 52, as best seen in FIG. 2C. Alternatively, two or more intersecting slits (not shown) may extend from the bottom 68 of the bore 62 to the second end 56 of the valve body 52, for example in an intersecting cross pattern (not shown). As best seen in FIG. 2A, the slit 64 may have a width at least as long as the diameter of the bore 62, and, optionally, may have a length greater than the diameter of the bore 62, e.g., between about 0.25 and eight millimeters (0.25-8 mm). The width of the slit 64 may be substantially uniform between the bottom 68 of the bore 62 and the second end 56 of the valve body 52 or may vary along its length. For example, the width of the slit 64 may be greater adjacent the bore 62 than at the second end 56 of the valve body 52, if desired.

The width of the slit 64 may be sufficient to allow opposing regions of the valve body 52 on either side of the slit 64 to be directed away from one another to accommodate receiving a medical device through the valve passage without substantial risk of the valve body 52 tearing uncontrollably. Thus, the valve body 52 may be resiliently flexible such that the opposing slit regions may expand when a medical device is inserted through the slit 64, yet resiliently close when the medical device is removed to maintain a substantially fluid tight seal through the valve passage 60. Alternatively, the slit 64 may provide a relatively small passage to guide a medical device from the bore 62 through the valve body 52 to the second end 56, e.g., with the slit 64 providing a preferential tear line through the valve body 52 as the medical device is inserted therethrough. In another alternative, the valve body 52 may include features, such as the recess 69, which may decrease or stop propagation of tears initiated as a medical device is inserted therethrough.

Optionally, the valve body 52 may include one or more additional features. For example, as shown in FIG. 2D, an alternative valve body 52' is shown that includes a first end 54' that is at least partially tapered, e.g., to facilitate introduction of a medical device therethrough and/or into the bore 62.' As shown, the first end 54' includes a surface 55' that tapers from the first end 54' into the bore 62,' which may guide a medical device introduced into a hub (not shown) towards the bore 62.' For example, if the medical device has a diameter smaller than the first hub lumen 32c (not shown, see, e.g., FIG. 3), the tapered surface 55' may facilitate guiding the medical device inserted into the first hub lumen 32c through the valve 50' and into the second hub lumen 34c.

In addition or alternatively, the valve body 52' may include a convex or otherwise shaped second end 56' that includes less material towards the outer surface 57' of the valve body 52' than towards the central longitudinal axis 18.' Such a shape may facilitate directing opposing slit regions away from one another when a medical device is inserted into the slit 64' since there is less material to bunch up adjacent the wall of the second hub portion 34 (not shown, see, e.g., FIG. 3). Such a shape may also distribute forces within the valve body 52' when a medical device is inserted into the slit 64' to prevent undesired tearing of the valve body 52.' Such a shape may also facilitate sealing the valve when no medical device is inserted into the slit 64' and pressure is applied to the second end 56.'

Returning to FIGS. 2A-2C, the valve body 52 may be formed from an elastomeric material, such as silicone, chronoprene, isoprene, santoprene, and the like. In one embodiment, the valve body 52 may be integrally formed as a single piece, e.g., by injection molding, casting, and the like. For example, with continued reference to FIG. 2A, the valve body 52, including connectors 58, bore 62, and recess 69, may be integrally formed, e.g., by injection molding, casting, and the like. Alternatively, the valve body 52 may be formed as a solid body and the connectors 58, bore 62, and/or recess 69 may be formed into the solid body, e.g., by cutting, machining, and the like.

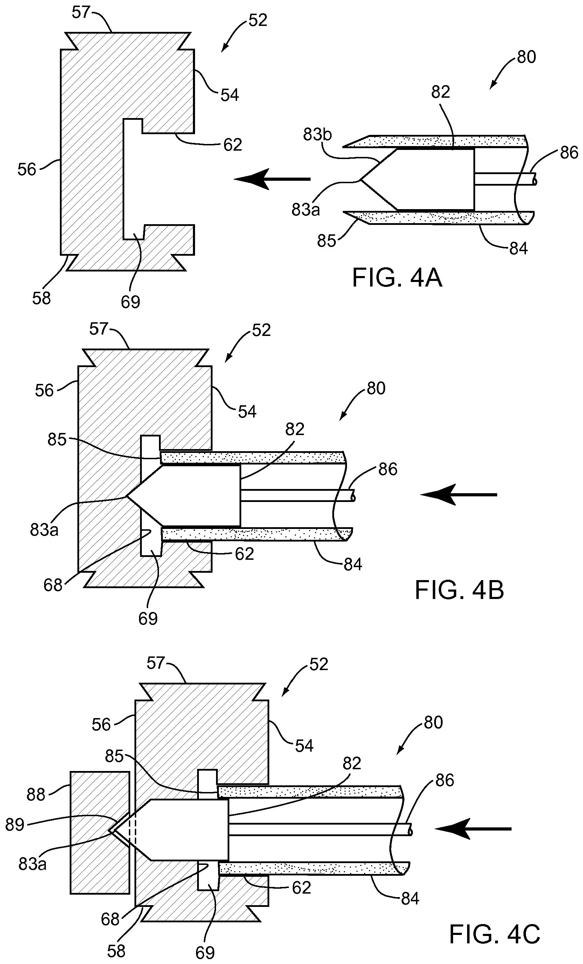

Once the valve body 52 including the bore 62 are formed, the slit 64 may be formed in the valve body 52, e.g., to complete the valve passage 60. Turning to FIGS. 4A-4C, an exemplary method is shown for forming the slit 64 in the valve body 52 of FIGS. 2A-2C. Initially, as shown, a valve body 52 may be provided including a bore 62 (and optionally recess 69) formed therein, as described above. A tool 80 may be provided to cut or otherwise penetrate through the valve body 52 to create the slit 64. For example, the tool 80 may include a blade or other cutting element 82 for mechanically cutting or slicing through the valve body 52, although alternatively other devices may be used, for example, a heated blade, wire, or other element that may melt through the valve body 52, e.g., in addition to or instead of mechanically cutting the valve body 52.

As shown in FIGS. 4A-4C, the tool 82 includes a blade or other mechanical cutting element 82, e.g., having a width corresponding the desired width of the slit 64 to be formed. In addition or alternatively, the cutting element 82 may have a relatively thin thickness, e.g., between about 0.05 and 0.75 millimeter (0.05-0.75 mm), to facilitate advancing the cutting element 82 through the material of the valve body 52 with minimal friction and/or risk of tearing the valve body 52. As shown, the cutting element 82 may include a sharpened tip 83a and/or one or more sharpened edges 83b (two shown), which may facilitate cutting into the material of the valve body 52.

Optionally, the cutting element 82 may be deployable from a sleeve or other assembly 84, e.g., such that the cutting element 82 may be initially provided within the sleeve 84 and may be advanced from and/or retracted into the sleeve 84 when desired. For example, the tool 80 may include a handle (not shown) coupled to a proximal end of the sleeve 84 that includes an actuator (also not shown) coupled to a shaft or other actuator member 86 carrying the cutting element 82.

Optionally, as shown in FIG. 4C, the tool 80 may include a rigid stop 88, which may be placed opposite the sleeve 84 and cutting element 82 to facilitate penetrating the cutting element 82 sufficiently into the valve body 52. For example, the stop 88 may include a slot or other recess 89 that may be sized to receive at least the tip 83a of the cutting element 82 therein, e.g., to ensure that the cutting element 82 penetrates a sufficient depth into or through the valve body, as described further below.

The sleeve 84 may include a distal end 85 which may be tapered, as shown in FIG. 4A, e.g., to provide a transition that facilitates advancing the sleeve 84 at least partially into the bore 62 of the valve body 52. Alternatively, the distal end 85 may be blunt, e.g., as shown in FIGS. 4B and 4C, or otherwise shaped. The distal end 85 of the sleeve 84 may have a diameter or other cross-section smaller than the bore 62 in its relaxed state, e.g., such that the distal end 85 may be inserted into the bore 62 without deforming the valve body 52, e.g., before deploying the cutting element 82. Alternatively, the distal end 85 of the sleeve 84 may be larger than the bore 62, e.g., such that the bore 62 may be expanded when the sleeve 84 is inserted therein, as shown in FIGS. 4B and 4C. In this alternative, the cutting element 82 may have a width greater than the diameter of the bore 62 in the relaxed state, e.g., to form a slit 64 having a width greater than the diameter of the bore 62.

Turning to FIG. 4A, the cutting element 82 is shown retracted within the sleeve 84 and the sleeve 84 is directed towards the valve body 50, i.e., into the bore 62. As shown in FIG. 4B, the distal end 85 of the sleeve 84 has been inserted into the bore 62, e.g., until the distal end 85 contacts or is immediately adjacent the bottom 68 of the bore 62. As shown, the bore 62 has been expanded by the distal end 85 of the sleeve 84 to a diameter approaching the diameter of the recess 69, although alternatively, the bore 62 may be expanded further, which may also expand the recess 69.

Turning to FIG. 4C, the cutting element 82 has been advanced from the sleeve 84 such that the distal tip 83a penetrates through the bottom 68 of the bore 62 and through the valve body 52 and at least partially out through the second end 56. Thus, the cutting element 82 has created a slit 64 that extends from the bottom 68 of the bore 62 through to the second end 56 of the valve body 52. As shown, the distal tip 83a of the cutting element 82 has exited the valve body 52 and entered the slot 89 in the stop 88. The stop 88 may also prevent deformation of the valve body 52 when the cutting element 82 is advanced through the second end 56, thereby reducing the risk of tearing or otherwise damaging the valve body 52.

The cutting element 82 may be retracted back into the sleeve 84 and the sleeve 84 removed from the bore 62, thereby providing a slit 64 extending through the valve body 52, as shown in FIGS. 2A-2C. In an alternative embodiment, the cutting element 82 or other tool may be used to create the slit 64 by penetrating the second end 56 of the valve body 52, e.g., through the bottom 68 of the bore 62, if desired, rather than through the bore 62 from the first end 54 of the valve body 52.

Optionally, lubricant or other material may be introduced into the bore 62, e.g., into the recess 69. Any additional features may be formed in the valve body 52, if desired, to create the final valve 50. The valve 50 may thereafter be incorporated into a hub or other apparatus.

Returning to FIGS. 1A and 1B, any of the valves herein, such as valve 50 or 50,' may be secured to a hub 30 of an apparatus 10, e.g., a sheath, catheter, or other tubular member, to accommodate receiving one or more devices, e.g., a catheter, lead, guidewire or other medical device (not shown), through the hub 30 and into the lumen 16 of the tubular body 11, while providing a substantially fluid-tight seal. For example, as described above, a hub 30 may be formed that includes a first hub portion 32 and a second hub portion 34 coupled together by hub arm 36 such that first and second hub lumens 32c, 34c of the hub portions 32, 34 are aligned with one another and the hub portions 32, 34 are spaced apart from one another to define a gap 35, as best seen in FIG. 1B.

A valve 50 may be selected and secured to the hub 30 within the gap 35. For example, using any of the methods described above, a valve 50 may be formed that includes a valve body 52 including a valve passage 60 extending therethrough. During manufacturing or final assembly, the valve body 52 may be secured within the gap 35 between the first and second hub portions 32, 34 such that the valve passage 60 is aligned with the hub lumens 32c, 34c, e.g., to accommodate receiving a medical device through the hub 30 into the tubular body 11 while providing a substantially fluid tight seal.

Alternatively, it may be possible to provide a plurality of valves to an end user separate from a hub 30 such that the user may select a desired valve for use during a particular procedure. For example, different valves having different configurations, e.g., different sizes of valve passage 50, different material properties, and the like, may be provided to a user, and the user may simply select a desired configuration from the available valves and secure the valve to the hub 30.

For example, as described above, the first and second hub portions 32, 34 may be directed away from one another, e.g., by bending the hub arm 36 slightly, to increate a length of the gap 35, thereby providing additional space to insert the valve body 52 between the hub portions 32, 34. In one embodiment, the hub arm 36 may be bent along the longitudinal axis 18, e.g., such that the first and second hub portions 32 remain generally aligned with one another but the distance between them is increased. In addition or alternatively, the hub arm 36 may be bent transversely relative to the longitudinal axis 18, e.g., such that the first and second hub portions 32 are directed off the longitudinal axis 18, e.g., on opposite sides.

The valve body 52 may then be inserted within the gap 35 between the hub portions 32, 34, and the hub portions 32, 34 may be directed back towards one another to capture the valve body 52 between the hub portions 32, 34. In one embodiment, the hub portions 32, 34 may be directed towards one another simply by releasing the hub arm 36 and/or the hub portions 32, 34, whereupon the hub arm 36 may resiliently return towards its original shape, thereby directing the hub portions 32, 34 towards one another.

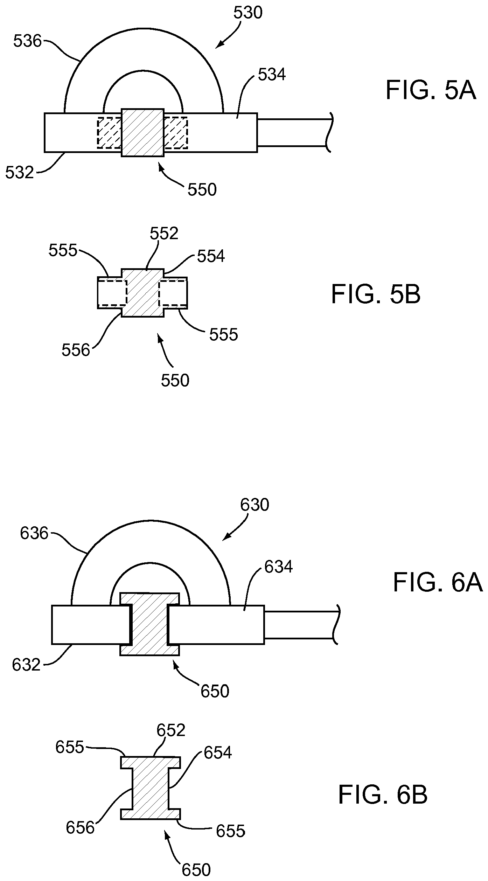

The resulting interference fit alone may be sufficient to secure the valve 50 within the gap 35. For example, as shown in FIG. 3, when the valve 50 is captured between the hub portions 32, 34, the beveled edges 32d, 34d may engage the slots 58 in the valve body 52, thereby substantially securing the valve 50 within the gap 35. In an alternative embodiment, shown in FIGS. 5A and 5B, a valve 550 may be provided that includes flanged connectors or tubular extensions 555 on one or both ends 554, 556 of a valve body 552, which may formed similar to any of the embodiments herein. As shown in FIG. 5B, the tubular extensions 555 may be "undersized," i.e., smaller than the corresponding hub portions 532, 534 between the valve 550 is secured, e.g., such that the tubular extensions 555 may be received in the lumens of the hub portions 532, 534. Alternatively, as shown in FIGS. 6A and 6B, a valve 650 may be provided that includes flanged connectors or tubular extensions 655 on one or both ends 654, 656 of a valve body 652 that may be "oversized," i.e., larger than corresponding hub portions 632, 634. Thus, in this alternative, the tubular extensions 655 may extend over a portion of the respective hub portions 632, 634 to provide an interference fit. In these alternatives, the tubular extensions 555, 655 may have lengths sufficient to substantially seal the valve 550, 650 between the hub portions, e.g., between about 0.5 and three millimeters (0.5-3 mm). In addition or alternatively, the tubular extensions 555, 655 may be attached to the hub portions, e.g., by bonding with adhesive, fusing, sonic welding, and the like.

If desired, the valve body 52 may in addition or alternatively be secured using other methods, e.g., at least one of bonding with adhesive, welding, insertion molding, or fusing the valve body 52 to the first and/or second hub portions 32, 34, surrounding bands (not shown), and/or other connectors. Where adhesive is used to secure the valve 50 within the gap 35, the valve body 52 and/or the hub portions 32, 34 may include recesses, channels, or other features (not shown) designed to receive and/or distribute adhesive at the interface between the hub portions 32, 34 and the valve body 52.

If the hub portions 32, 34 include relatively thin walled regions and/or the valve 50 includes a valve passage offset towards one side of the valve body 52, one or more connectors or alignment features may be provided on the valve 50 and/or hub portions 32, 34 to ensure that the valve 50 is properly oriented when captured within the gap 35. For example, one of the hub portions 32, 34 and the valve body 52 may include one or more tabs (not shown) that may be received in corresponding one or more slots (also not shown) in the other of the hub portions 32, 34 and the valve body 52 only when the valve 50 is oriented properly relative to the hub portions 32, 34. Such alignment features may ensure that relatively thin walled regions of the hub portions 32, 34 are aligned with the thinner region of the valve body 52, which may facilitate slitting the hub 30 after use during a procedure, as described further below.

In an alternative embodiment, one of the first and second hub portions 32, 34 may be provided separate from the hub arm 36, e.g., during manufacturing, and the valve 50 may be secured to the hub 30 before attaching the separate hub portion. For example, with reference to FIG. 1B, the hub 30 may be formed with only the second hub portion 34 and the side port 38 attached to the hub arm 36. The valve body 52 may be secured to the first end 34a of the second hub portion 34, e.g., by bonding with adhesive, welding or fusing, one or more connectors, and the like. The first hub portion 32 may then be attached to the hub 30 and/or the valve 50. For example, the first hub portion 32 may be attached to the first end 36a of the hub arm 36, e.g., using the methods described above, and the second end 32b of the first hub portion 32 may be attached to the valve body 52, e.g., similar to the second hub portion 34.

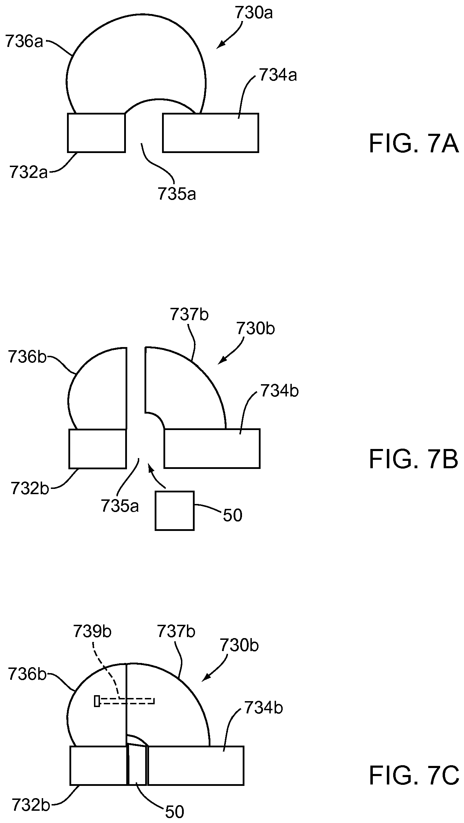

Turning to FIG. 7A, an alternative embodiment of a hub 730a is shown that includes a first hub portion 732a integrally formed with or otherwise attached to a hub arm 736a. A separate second hub portion 734a may be formed that may be attached to the hub arm 736a after a valve (not shown) has been attached to one or both hub portions 732a, 734a.

Turning to FIGS. 7B and 7C, yet another alternative embodiment of a hub 730b is shown that includes a first hub portion 732b integrally formed with or attached to a first handle portion 736b and a second hub portion 734b integrally formed with or attached to a second handle portion 737b, as shown in FIG. 7B. When the handle portions 736b, 737b are placed together, as shown in FIG. 7C, a gap 735b remains between the hub portions 732b, 734b for receiving a valve 50, similar to other embodiments herein. Thus, when a valve 50 is secured to one or both of the hub portions 732b, 734b, the handle portions 736b, 737b may be attached together, e.g., using one or more fasteners 739b, e.g., screws, bolts, pins, rivets, and the like, as shown. In addition or alternatively, the handle portions 736b, 737b may be attached together by bonding with adhesive, fusing, sonic welding, cooperating detents or other connectors, and the like (not shown).

Once the valve 50 is secured or otherwise captured within the gap 35 of the hub 30, the hub 30 (which may be any of the embodiments herein) may be incorporated into an apparatus 10, as shown in FIG. 1A. For example, the second end 34b of the second hub portion 34 may be attached to the proximal end 12 of the tubular body 11, e.g., after (or optionally before) assembling the hub 30. The resulting apparatus 10 may be sterilized and/or packaged, as desired, and provide to a user, or the apparatus 10 without the valve 50 may be provided to a user for assembly immediately before a procedure, e.g., as described above.

During use, the apparatus 10 may be used for delivering a medical device into a body lumen within a patient's body, e.g., a lead, catheter, and the like, into a patient's vasculature or other body lumen, as described above. For example, a distal end 14 of the tubular body 11 may be introduced into a patient's vasculature with the hub 30 and valve 50 remaining outside the patient's body. The tubular body 11 may be advanced through the patient's vasculature, e.g., to position the distal end 14 and a desired location, e.g., a coronary vein within the patient's heart or other body lumen. A medical device, e.g., a pacing or other electrical lead (not shown), may be inserted through the first hub portion 32, the valve 50, and the second hub portion 34 and into the tubular body 11 until a distal end of the medical device is advanced into the body lumen, e.g., exiting or remaining within the distal end 14 of the tubular body 11.

The apparatus 10 may then be removed to leave the medical device implanted within the patient's body. The configuration of the hub 30 may facilitate removing the apparatus 10 from around the medical device without substantial risk of dislodging or otherwise moving the medical device. For example, cardiac leads often include relatively large proximal hubs, e.g., including electrical connectors and the like, which may prevent the apparatus 10 from being removed over the hub. Instead, a slitter or other tool (not shown) may be used to slit the hub 30, valve, and tubular body 11 to open the apparatus 10 and allow easy removal despite a large hub or other obstacle.

For example, a slitter may be used sequentially cut the first hub portion 32, the valve body 52, the second hub portion 34, and the tubular body 11. If the hub portions 32, 34 and/or valve body 52 include relatively thin walled regions aligned with one another, the regions may be identified, e.g., by a colored line, a recess (not shown) in the first end 32a of the first hub portion 32, and the like, to facilitate identification by the user. Optionally, the tubular body 11 may include a tear-away or other weakened region that may be aligned with the relatively thin walled regions of the hub 30, which may facilitate slitting or may simply propagate separation of the region along the length of the tubular body 11 with or without use of a slitter or other tool.

Because the valve body 52 is slit separately from the hub portions 32, 34, the user only needs to slit one layer of material, which may facilitate slitting the hub 30, e.g., compared to hubs with valves disposed concentrically within a tubular hub. Simultaneously slitting such a concentric valve and tubular hub may require greater force, since multiple layers of dissimilar materials must be slit together, which increases the risk of moving the medical device being implanted.

With reference to FIGS. 2A-2C, another advantage of the valves described herein is that the valve body 52 may have an overall length that is substantially shorter than conventional valves. This relatively short length may reduce friction between the valve 50 and medical device inserted through the valve passage 60 since there is less surface area to contact the medical device. This is particularly useful when the valve body 52 is formed from materials, such as silicone, which may be tacky.

With further reference to FIGS. 2A-2C, still another advantage of the valves described herein is that the recess 69 at the bottom of the bore 62 within the valve body 52 may limit propagation of a tear when a medical device is inserted through the valve passage 60 of the valve 50. For example, if a medical device is inserted through the bore 62 into the slit 64 and causes the slit 64 to tear further into the valve body 52, the recess 69 may provide a negative cavity creating a "rip stop" that may prevent the tear from propagating towards the first end 54 of the valve body 52. If the valve body 52 surrounding the bore 62 were to tear, there would be substantially increased risk of leaking around the medical device inserted through the valve passage 60. Similarly, any tear that is created by over-expansion of the bore 62 may be prevented from propagating towards the valve body 52 surrounding the slit 64 due to the rip stop provided by the recess 69.

With further reference to FIGS. 2A-2C, still another advantage of valves described herein is that because such valves are not disposed concentrically within a tubular hub, they may expand unconstrained to accommodate insertion of a medical device. As a result, the valve 50, including the bore 62, may be sized to seal on a relatively small device, such as a guidewire, while accommodating passage of a relatively larger device, such as a lead or catheter, without causing excessive friction during passage of the relatively larger device.

The foregoing disclosure of the exemplary embodiments has been presented for purposes of illustration and description. It is not intended to be exhaustive or to limit the invention to the precise forms disclosed. Many variations and modifications of the embodiments described herein will be apparent to one of ordinary skill in the art in light of the above disclosure.

Further, in describing representative embodiments, the specification may have presented the method and/or process as a particular sequence of steps. However, to the extent that the method or process does not rely on the particular order of steps set forth herein, the method or process should not be limited to the particular sequence of steps described. As one of ordinary skill in the art would appreciate, other sequences of steps may be possible. Therefore, the particular order of the steps set forth in the specification should not be construed as limitations on the claims.

While the invention is susceptible to various modifications, and alternative forms, specific examples thereof have been shown in the drawings and are herein described in detail. It should be understood, however, that the invention is not to be limited to the particular forms or methods disclosed, but to the contrary, the invention is to cover all modifications, equivalents and alternatives falling within the scope of the appended claims.

## Claims

1. A hub (30, 530, 630, 730, 830, 930, 1130) for a sheath, catheter, or other tubular device (10), comprising a first hub portion (32, 532, 632, 732, 832, 932, 1132) having a first end (32a), a second end (32b) and a first hub lumen (32c) extending therebetween, the first hub lumen being sized for receiving a medical device therethrough and extending concentrically around a longitudinal axis (18) of the hub (30);
a second hub portion (34, 534, 634, 734, 834, 934, 1134) having a first end (34a), a second end (34b) and a second hub lumen (34c) extending therebetween, the second hub portion coupled to the first hub portion such that the first and second hub lumens are aligned with one another along the longitudinal axis and the second end (32b) of the first hub portion and the first end (34a) of the second hub portion are spaced apart from one another to define a gap (35, 735, 835, 935, 1135);
a hub arm (36, 536, 636, 736, 836, 936, 1136) including a first end (36a) attached to the first hub portion (32, 532, 632, 732, 832, 932, 1132) and a second end (36b) attached to the second hub portion (34, 534, 634, 734, 834, 934, 1134), thereby maintaining the second end (32b) of the first hub portion and the first end (34a) of the second hub portion spaced apart from one another to define the gap (35, 735, 835, 935, 1135); and
a valve (50, 550, 650) secured within the gap between the second end (32b) of the first hub portion and the first end (34a) of the second hub portion, the valve comprising a valve passage (60) extending therethrough configured to accommodate receiving a medical device therethrough when the medical device is introduced through the first and second hub lumens of the first and second hub portions.

2. The hub of any preceding claim, wherein at least one of the first hub portion, the second hub portion, and the valve comprises one or more connectors (32d, 34d, 58, 555, 655) for securing the valve within the gap.

3. The hub of claim 2, wherein the one or more connectors comprise tubular extensions (555, 655) that extend from the valve body and are aligned with the valve passage, the tubular extensions configured to engage the ends of the first and second hub portions adjacent the gap.

4. The hub of claim 3, wherein the tubular extensions comprise one of annular flanges (655) that extend around an exterior of the ends of the first and second hub portions or annular flanges (555) that are sized to be received in the lumens of the first and second hub portions.

5. The hub of any preceding claim, wherein the valve is secured to the first and second hub portions by at least one of an adhesive, one or more cooperating connectors, and a fused seam.

6. The hub of any preceding claim, wherein the valve comprises an elastomeric material.

7. The hub of any preceding claim, wherein the valve passage comprises a bore (62) extending partially from a first end (54) of the valve towards a second end (56) of the valve, the bore extending substantially parallel to the longitudinal axis, the valve being resiliently flexible such that a cross-section of the bore may be increased to accommodate receiving a medical device through the bore that has a cross-section larger than the bore while maintaining a substantially fluid tight seal around the medical device.

8. The hub of claim 7, wherein the bore defines a central axis (66) that is substantially parallel to and offset from the longitudinal axis such that one side of the bore is closer to an outer surface of the valve than the opposite side of the bore.

9. The hub of claim 8, wherein one or both of the first and second hub portions comprise a relatively thin walled region aligned with the one side of the bore closer to the outer surface of the valve to facilitate slitting through one or both of the first and second hub portions and the valve along the relatively thin walled region and the one side of the bore.

10. A method for making a sheath, catheter, or other tubular member, comprising:
forming a hub (30, 530, 630, 730, 830, 930, 1130) comprising a first hub portion (32, 532, 632, 732, 832, 932, 1132) having a first end (32a), a second end (32b) and a first hub lumen (32c) extending therebetween, a second hub portion (34, 534, 634, 734, 834, 934, 1134) having a first end (34a), a second end (34b) and a second hub lumen (34c) extending therebetween, and a hub arm (36, 536, 636, 736, 836, 936, 1136), the second hub portion coupled to the first hub portion such that the first and second hub lumens are aligned with one another and the second end (32b) of the first hub portion and the first end (34a) of the second hub portion are spaced apart from one another to define a gap (35, 735, 835, 935, 1135), the hub arm including a first end (36a) attached to the first hub portion (32, 532, 632, 732, 832, 932, 1132) and a second end (36b) attached to the second hub portion (34, 534, 634, 734, 834, 934, 1134), thereby maintaining the second end (32b) of the first hub portion and the first end (34a) of the second hub portion spaced apart from one another to define the gap (35, 735, 835, 935, 1135);
selecting a valve body (50, 550, 650) comprising a valve passage (60) extending therethrough; and
securing the valve body within the gap between the second end (32b) of the first hub portion and the first end (34a) of the second hub portion such that the valve passage accommodates receiving a medical device therethrough when the medical device is introduced through the first and second hub lumens of the first and second hub portions while providing a substantially fluid tight seal.

11. The method of claim 10, wherein securing the valve body within the gap comprises:
directing the first (832, 932, 1132) and second hub portions (834, 934, 1134) away from one another to increase a size of the gap (835, 935, 1135);
inserting the valve body (50) within the gap between the first and second hub portions; and
directing the first and second hub portions towards one another to capture the valve body between the first and second hub portions.

12. The method of claim 11, wherein directing the first and second hub portions away from one another comprises bending the hub arm.

13. The method of claim 10 or 11, wherein securing the valve body within the gap comprises:
securing the valve body to the second hub portion before the first hub portion is attached to the hub;
attaching the first hub portion to the hub; and
securing the valve body to the first hub portion.

14. The method of claim 13, wherein the securing the valve body comprises at least one of bonding and fusing the valve body to the second hub portion.

15. The method of claim 11, wherein forming the hub comprises integrally molding the first and second hub portions as a single piece, and
wherein the hub includes a bridge piece (939, 1139) extending between the first and handle portions adjacent the gap, the method further comprising:
severing the bridge piece before directing the first and second hub portions away from one another to create opposing stops, and
wherein the first and second hub portions are directed towards one another to capture the valve body until the opposing stops contact one another.

16. The method of claim 15, wherein the valve body is secured within the gap by one or more fasteners (939b) inserted through the stops to fix the first and second handle portions relative to one another.

## Patentansprüche

1. Nabe (30, 530, 630, 730, 830, 930, 1130) für eine Hülse, einen Katheter oder eine andere röhrenförmige Vorrichtung (10), umfassend
einen ersten Nabenabschnitt (32, 532, 632, 732, 832, 932, 1132), welcher ein erstes Ende (32a), ein zweites Ende (32b) und ein sich dazwischen erstreckendes erstes Nabenlumen (32c) aufweist, wobei das erste Nabenlumen zum Aufnehmen einer medizinischen Vorrichtung dadurch bemessen ist und sich konzentrisch um eine longitudinale Achse (18) der Nabe (30) erstreckt;
einen zweiten Nabenabschnitt (34, 534, 634, 734, 834, 934, 1134), welcher ein erstes Ende (34a), ein zweites Ende (34b) und ein sich dazwischen erstreckendes zweites Nabenlumen (34c) aufweist, wobei der zweite Nabenabschnitt derart mit dem ersten Nabenabschnitt gekoppelt ist, dass das erste und das zweite Nabenlumen entlang der longitudinalen Achse zueinander ausgerichtet sind und das zweite Ende (32b) des ersten Nabenabschnitts und das erste Ende (34a) des zweiten Nabenabschnitts voneinander beabstandet sind, um eine Lücke (35, 735, 835, 935, 1135) zu definieren;
einen Nabenarm (36, 536, 636, 736, 836, 936, 1136), welcher ein an dem ersten Nabenabschnitt (32, 532, 632, 732, 832, 932, 1132) angebrachtes erstes Ende (36a) und ein an dem zweiten Nabenabschnitt (34, 534, 634, 734, 834, 934, 1134) angebrachtes zweites Ende (36b) umfasst, wodurch das zweite Ende (32b) des ersten Nabenabschnitts und das erste Ende (34a) des zweiten Nabenabschnitts voneinander beabstandet bleiben, um die Lücke (35, 735, 835, 935, 1135) zu definieren; und
ein Ventil (50, 550, 650), welches in der Lücke zwischen dem zweiten Ende (32b) des ersten Nabenabschnitts und dem ersten Ende (34a) des zweiten Nabenabschnitts gesichert ist, wobei das Ventil einen sich dadurch erstreckenden Ventildurchgang (60) umfasst, welcher dazu eingerichtet ist, eine Aufnahme einer medizinischen Vorrichtung dadurch unterzubringen, wenn die medizinische Vorrichtung durch das erste und das zweite Nabenlumen des ersten und des zweiten Nabenabschnitts eingeführt ist.

2. Nabe nach einem vorhergehenden Anspruch, wobei wenigstens eines des ersten Nabenabschnitts, des zweiten Nabenabschnitts und des Ventils einen oder mehrere Verbindungselemente (32d, 34d, 58, 555, 655) zum Sichern des Ventils in der Lücke umfasst.

3. Nabe nach Anspruch 2, wobei das eine oder die mehreren Verbindungselemente röhrenförmige Verlängerungen (555, 655) umfassen, welche sich von dem Ventilkörper erstrecken und zu dem Ventildurchgang ausgerichtet sind, wobei die röhrenförmigen Verlängerungen dazu eingerichtet sind, die Enden des ersten und des zweiten Nabenabschnitts benachbart der Lücke in Eingriff zu nehmen.

4. Nabe nach Anspruch 3, wobei die röhrenförmigen Verlängerungen eines aus ringförmigen Flanschen (655), welche sich um ein Äußeres der Enden des ersten und des zweiten Nabenabschnitts herum erstrecken, oder ringförmigen Flanschen (555) umfassen, welche bemessen sind, um in den Lumen des ersten und des zweiten Nabenabschnitts aufgenommen zu sein.

5. Nabe nach einem vorhergehenden Anspruch, wobei das Ventil durch wenigstens eines aus einem Klebstoff, einem oder mehreren zusammenwirkenden Verbindungselementen und einer verschmolzenen Naht an dem ersten und dem zweiten Nabenabschnitt gesichert ist.

6. Nabe nach einem vorhergehenden Anspruch, wobei das Ventil ein elastomeres Material umfasst.

7. Nabe nach einem vorhergehenden Anspruch, wobei der Ventildurchgang eine Bohrung (62) umfasst, welche sich teilweise von einem ersten Ende (54) des Ventils in Richtung eines zweiten Endes (56) des Ventils erstreckt, wobei sich die Bohrung im Wesentlichen parallel zu der longitudinalen Achse erstreckt, wobei das Ventil elastisch flexibel ist, sodass ein Querschnitt der Bohrung vergrößert werden kann, um eine Aufnahme einer medizinischen Vorrichtung durch die Bohrung unterzubringen, welche einen Querschnitt aufweist, welcher größer ist als die Bohrung, während eine im Wesentlichen fluiddichte Dichtung um die medizinische Vorrichtung herum beibehalten wird.

8. Nabe nach Anspruch 7, wobei die Bohrung eine zentrale Achse (66) definiert, welche im Wesentlichen parallel zu und versetzt von der longitudinalen Achse ist, sodass eine Seite der Bohrung näher an einer äußeren Fläche des Ventils ist als die gegenüberliegende Seite der Bohrung.

9. Nabe nach Anspruch 8, wobei einer oder beide des ersten und des zweiten Nabenabschnitts einen relativ dünnwandigen Bereich umfassen, welcher zu der einen Seite der Bohrung ausgerichtet ist, welche näher an der äußeren Fläche des Ventils ist, um ein Durchschlitzen durch einen oder beide des ersten und des zweiten Nabenabschnitts und des Ventils entlang des relativ dünnwandigen Bereichs und der einen Seite der Bohrung zu erleichtern.

10. Verfahren zur Herstellung einer Hülse, eines Katheters oder eines anderen röhrenförmigen Elements, umfassend:
Bilden einer Nabe (30, 530, 630, 730, 830, 930, 1130), welche einen ersten Nabenabschnitt (32, 532, 632, 732, 832, 932, 1132), welcher ein erstes Ende (32a), ein zweites Ende (32b) und ein sich dazwischen erstreckendes erstes Nabenlumen (32c) aufweist, einen zweiten Nabenabschnitt (34, 534, 634, 734, 834, 934, 1134), welcher ein erstes Ende (34a), ein zweites Ende (34b) und ein sich dazwischen erstreckendes zweites Nabenlumen (34c) aufweist, und einen Nabenarm (36, 536, 636, 736, 836, 936, 1136) umfasst, wobei der zweite Nabenabschnitt derart mit dem ersten Nabenabschnitt gekoppelt ist, dass das erste und das zweite Nabenlumen zueinander ausgerichtet sind und das zweite Ende (32b) des ersten Nabenabschnitts und das erste Ende (34a) des zweiten Nabenabschnitts voneinander beabstandet sind, um eine Lücke (35, 735, 835, 935, 1135) zu definieren, wobei der Nabenarm ein an dem ersten Nabenabschnitt (32, 532, 632, 732, 832, 932, 1132) angebrachtes erstes Ende (36a) und ein an dem zweiten Nabenabschnitt (34, 534, 634, 734, 834, 934, 1134) angebrachtes zweites Ende (36b) umfasst, wodurch das zweite Ende (32b) des ersten Nabenabschnitts und das erste Ende (34a) des zweiten Nabenabschnitts voneinander beabstandet bleiben, um die Lücke (35, 735, 835, 935, 1135) zu definieren;
Auswählen eines Ventilkörpers (50, 550, 650), welcher einen sich dadurch erstreckenden Ventildurchgang (60) umfasst; und
Sichern des Ventilkörpers in der Lücke zwischen dem zweiten Ende (32b) des ersten Nabenabschnitts und dem ersten Ende (34a) des zweiten Nabenabschnitts, sodass der Ventildurchgang eine Aufnahme einer medizinischen Vorrichtung dadurch unterbringt, wenn die medizinische Vorrichtung durch das erste und das zweite Nabenlumen des ersten und des zweiten Nabenabschnitts eingeführt wird, während eine im Wesentlichen fluiddichte Dichtung bereitgestellt wird.

11. Verfahren nach Anspruch 10, wobei das Sichern des Ventilkörpers in der Lücke umfasst:
Lenken des ersten (832, 932, 1132) und des zweiten Nabenabschnitts (834, 934, 1134) voneinander weg, um eine Größe der Lücke (835, 935, 1135) zu vergrößern;
Einführen des Ventilkörpers (50) in die Lücke zwischen dem ersten und dem zweiten Nabenabschnitt; und
Lenken des ersten und des zweiten Nabenabschnitts zueinander hin, um den Ventilkörper zwischen dem ersten und dem zweiten Nabenabschnitt zu fangen.

12. Verfahren nach Anspruch 11, wobei das Lenken des ersten und des zweiten Nabenabschnitts voneinander weg ein Biegen des Nabenarms umfasst.

13. Verfahren nach Anspruch 10 oder 11, wobei das Sichern des Ventilkörpers in der Lücke umfasst:
Sichern des Ventilkörpers an dem zweiten Nabenabschnitt, bevor der erste Nabenabschnitt an der Nabe angebracht wird;
Anbringen des ersten Nabenabschnitts an der Nabe; und
Sichern des Ventilkörpers an dem ersten Nabenabschnitt.

14. Verfahren nach Anspruch 13, wobei das Sichern des Ventilkörpers wenigstens eines aus einem Verbinden und einem Verschmelzen des Ventilkörpers mit dem zweiten Nabenabschnitt umfasst.

15. Verfahren nach Anspruch 11, wobei das Bilden der Nabe ein integrales Formen des ersten und des zweiten Nabenabschnitts als ein einzelnes Stück umfasst und
wobei die Nabe ein Brückenstück (939, 1139) umfasst, welches sich zwischen dem ersten und dem zweiten Abschnitt benachbart der Lücke erstreckt, wobei das Verfahren ferner umfasst:
Durchtrennen des Brückenstücks vor dem Lenken des ersten und des zweiten Nabenabschnitts voneinander weg, um gegenüberliegende Anschläge zu erzeugen, und
wobei der erste und der zweite Nabenabschnitt zueinander hin gelenkt werden, um den Ventilkörper zu fangen, bis die gegenüberliegenden Anschläge einander berühren.

16. Verfahren nach Anspruch 15, wobei der Ventilkörper in der Lücke durch ein oder mehrere Befestigungsmittel (939b) gesichert ist, welche durch die Anschläge eingeführt werden, um den ersten und den zweiten Griffabschnitt relativ zueinander zu befestigen.

## Revendications

1. Raccord (30, 530, 630, 730, 830, 930, 1130) pour une gaine, un cathéter ou un autre dispositif tubulaire (10), comprenant une première partie de raccord (32, 532, 632, 732, 832, 932, 1132) ayant une première extrémité (32a), une deuxième extrémité (32b) et une première lumière de raccord (32c) s'étendant entre elles, la première lumière de raccord étant dimensionnée pour recevoir un dispositif médical à travers celle-ci et s'étendant de manière concentrique autour d'un axe longitudinal (18) du raccord (30) ;
une deuxième partie de raccord (34, 534, 634, 734, 834, 934, 1134) ayant une première extrémité (34a), une deuxième extrémité (34b) et une deuxième lumière de raccord (34c) s'étendant entre elles, la deuxième partie de raccord étant couplée à la première partie de raccord de sorte que les première et deuxième lumières de raccord soient alignées entre elles le long de l'axe longitudinal et la deuxième extrémité (32b) de la première partie de raccord et la première extrémité (34a) de la deuxième partie de raccord sont espacées l'une par rapport à l'autre pour définir un espace (35, 735, 835, 935, 1135) ;
un bras de raccord (36, 536, 636, 736, 836, 936, 1136) comportant une première extrémité (36a) attachée à la première partie de raccord (32, 532, 632, 732, 832, 932, 1132) et une deuxième extrémité (36b) attachée à la deuxième partie de raccord (34, 534, 634, 734, 834, 934, 1134), maintenant ainsi la deuxième extrémité (32b) de la première partie de raccord et la première extrémité (34a) de la deuxième partie de raccord espacées l'une par rapport à l'autre pour définir l'espace (35, 735, 835, 935, 1135) ; et
une valve (50, 550, 650) fixée dans l'espace entre la deuxième extrémité (32b) de la première partie de raccord et la première extrémité (34a) de la deuxième partie de raccord, la valve comprenant un passage de valve (60) s'étendant à travers celle-ci configuré pour permettre la réception d'un dispositif médical à travers celui-ci lorsque le dispositif médical est introduit à travers les première et deuxième lumières de raccord des première et deuxième parties de raccord.

2. Raccord de l'une des revendications précédentes, dans lequel au moins l'une parmi la première partie de raccord, la deuxième partie de raccord et la valve comprend un ou plusieurs connecteur(s) (32d, 34d, 58, 555, 655) pour fixer la valve dans l'espace.

3. Raccord de la revendication 2, dans lequel le ou les plusieurs connecteur(s) comprend/comprennent des extensions tubulaires (555, 655) qui s'étendent depuis le corps de valve et sont alignées avec le passage de valve, les extensions tubulaires étant configurées pour s'engager avec les extrémités des première et deuxième parties de raccord adjacentes à l'espace.

4. Raccord de la revendication 3, dans lequel les extensions tubulaires comprennent l'une parmi des brides annulaires (655) qui s'étendent autour de l'extérieur des extrémités des première et deuxième parties de raccord et des brides annulaires (555) qui sont dimensionnées pour être reçues dans les lumières des première et deuxième parties de raccord.

5. Raccord de l'une des revendications précédentes, dans lequel la valve est fixée aux première et deuxième parties de raccord par au moins l'un(e) d'un adhésif, d'un ou de plusieurs connecteur(s) coopérant(s) et d'une couture soudée.

6. Raccord de l'une des revendications précédentes, dans lequel la valve comprend un matériau élastomère.

7. Raccord de l'une des revendications précédentes, dans lequel le passage de valve comprend un alésage (62) s'étendant partiellement d'une première extrémité (54) de la valve vers une deuxième extrémité (56) de la valve, l'alésage s'étendant sensiblement parallèlement à l'axe longitudinal, la valve étant élastiquement flexible de sorte qu'une section transversale de l'alésage puisse être agrandie pour permettre la réception d'un dispositif médical à travers l'alésage qui a une section transversale plus grande que l'alésage tout en maintenant un joint sensiblement étanche aux fluides autour du dispositif médical.

8. Raccord de la revendication 7, dans lequel l'alésage définit un axe central (66) qui est sensiblement parallèle à l'axe longitudinal et décalé par rapport à celui-ci, de sorte qu'un côté de l'alésage soit plus proche d'une surface extérieure de la valve que le côté opposé de l'alésage.

9. Raccord de la revendication 8, dans lequel l'une et/ou l'autre des première et deuxième parties de raccord comprend/comprennent une région à paroi relativement mince alignée avec le côté de l'alésage plus proche de la surface extérieure de la valve pour faciliter le fendage à travers l'une et/ou l'autre des première et deuxième parties de raccord et la valve le long de la région à paroi relativement mince et le côté de l'alésage.

10. Procédé de fabrication d'une gaine, d'un cathéter ou d'un autre élément tubulaire comprenant le fait :
de former un raccord (30, 530, 630, 730, 830, 930, 1130) comprenant une première partie de raccord (32, 532, 632, 732, 832, 932, 1132) ayant une première extrémité (32a), une deuxième extrémité (32b) et une première lumière de raccord (32c) s'étendant entre elles, une deuxième partie de raccord (34, 534, 634, 734, 834, 934, 1134) ayant une première extrémité (34a), une deuxième extrémité (34b) et une deuxième lumière de raccord (34c) s'étendant entre elles, et un bras de raccord (36, 536, 636, 736, 836, 936, 1136), la deuxième partie de raccord étant couplée à la première partie de raccord de sorte que les première et deuxième lumières de raccord soient alignées l'une par rapport à l'autre et la deuxième extrémité (32b) de la première partie de raccord et la première extrémité (34a) de la deuxième partie de raccord sont espacées l'une de l'autre pour définir un espace (35, 735, 835, 935, 1135), le bras de raccord comportant une première extrémité (36a) attachée à la première partie de raccord (32, 532, 632, 732, 832, 932, 1132) et une deuxième extrémité (36b) attachée à la deuxième partie de raccord (34, 534, 634, 734, 834, 934, 1134), maintenant ainsi la deuxième extrémité (32b) de la première partie de raccord et la première extrémité (34a) de la deuxième partie de raccord espacées l'une par rapport à l'autre pour définir l'espace (35, 735, 835, 935, 1135) ;
de sélectionner un corps de valve (50, 550, 650) comprenant un passage de valve (60) s'étendant à travers celui-ci ; et
de fixer le corps de valve dans l'espace entre la deuxième extrémité (32b) de la première partie de raccord et la première extrémité (34a) de la deuxième partie de raccord de sorte que le passage de valve permette la réception d'un dispositif médical à travers celui-ci lorsque le dispositif médical est introduit à travers les première et deuxième lumières de raccord des première et deuxième parties de raccord tout en fournissant un joint sensiblement étanche aux fluides.

11. Procédé de la revendication 10, dans lequel la fixation du corps de valve dans l'espace comprend le fait :
d'éloigner les première (832, 932, 1132) et deuxième (834, 934, 1134) parties de raccord l'une de l'autre pour augmenter la taille de l'espace (835, 935, 1135) ;
d'insérer le corps de valve (50) dans l'espace entre les première et deuxième parties de raccord ; et
de rapprocher les première et deuxième parties de raccord l'une de l'autre pour serrer le corps de valve entre les première et deuxième parties de raccord.

12. Procédé de la revendication 11, dans lequel le fait d'éloigner les première et deuxième parties de raccord l'une de l'autre comprend le fait de plier le bras de raccord.

13. Procédé de la revendication 10 ou 11, dans lequel la fixation du corps de valve dans l'espace comprend le fait :
de fixer le corps de valve à la deuxième partie de raccord avant que la première partie de raccord ne soit attachée au raccord ;
d'attacher la première partie de raccord au raccord ; et
de fixer le corps de valve à la première partie de raccord.

14. Procédé de la revendication 13, dans lequel la fixation du corps de valve comprend au moins l'une de la liaison et de la fusion du corps de valve à/avec la deuxième partie de raccord.

15. Procédé de la revendication 11, dans lequel la formation du raccord comprend le moulage d'un seul tenant des première et deuxième parties de raccord en une seule pièce, et
dans lequel le raccord comporte une pièce en pont (939, 1139) s'étendant entre les première et deuxième parties de poignée adjacentes à l'espace, le procédé comprenant en outre le fait :
de sectionner la pièce en pont avant d'éloigner les première et deuxième parties de raccord l'une de l'autre pour créer des butées opposés, et
dans lequel les première et deuxième parties de raccord sont rapprochées l'une de l'autre pour serrer le corps de valve jusqu'à ce que les butées opposées entrent en contact l'une avec l'autre.

16. Procédé de la revendication 15, dans lequel le corps de valve est fixé dans l'espace par une ou plusieurs attache(s) (939b) insérée(s) à travers les butées pour fixer les première et deuxième parties de poignée l'une par rapport à l'autre.
